# EUROPEAN PATENT SPECIFICATION

(11) **EP 2 515 653 B1**
(45) Date of publication and mention of the grant of the patent: **09.09.2015**
(21) Application number: 10840152.2
(22) Date of filing: 23.12.2010
(51) Int. Cl.: A61K 9/20

(54) **PHARMACEUTICAL COMPOSITIONS FOR DETERRING MISUSE, ABUSE, AND DIVERSION**
PHARMAZEUTISCHE ZUSAMMENSETZUNGEN ZUR VORBEUGUNG DER UNSACHGEMÄSSEN, MISSBRÄUCHLICHEN UND ZWECKENTFREMDETEN VERWENDUNG
COMPOSITIONS PHARMACEUTIQUES POUR PRÉVENIR UN MAUVAISE USAGE, UN USAGE ABUSIF ET UN USAGE DÉTOURNÉ

(30) Priority: 24.12.2009 US 290059 P
(43) Date of publication of application: 31.10.2012
(73) Proprietor: Acura Pharmaceuticals, Inc., Palatine, IL 60067 (US)
(72) Inventor: BRZECZKO, Albert, Walter, Baltimore MD 21210 (US)
(74) Representative: Vossius & Partner Patentanwälte Rechtsanwälte mbB
(86) International application number: PCT/US2010/061974
(87) International publication number: WO 2011/079248

(56) References cited:
- US-A1- 2004 006 072
- US-A1- 2004 006 072
- US-A1- 2006 229 226
- US-A1- 2006 229 226
- US-A1- 2007 231 268
- US-A1- 2007 231 268
- US-A1- 2008 311 197
- US-A1- 2008 311 197
- US-A1- 2008 311 205
- US-A1- 2008 311 205

## Description

### BACKGROUND

Drug abusers may attempt to manipulate an oral solid pharmaceutical dosage unit containing one or more drugs susceptible to abuse by intentionally crushing, shearing, grinding, chewing, dissolving, heating, extracting or otherwise tampering with or damaging the dosage unit so that a significant portion or even the entire amount of the active drug(s) in the dosage unit become available for misuse, abuse and diversion. There are numerous methods a prospective abuser may employ to abuse a pharmaceutical dosage unit including but not limited to ( a) intravenous (I.V.) injection of dissolved dosage units), (b) nasal snorting of crushed dosage units, and (c) oral swallowing excess quantities of dosage units. One method of abuse of oral solid pharmaceutical dosage units involves extraction of the active abuseable drug(s) from the dosage unit by first mixing and dissolving the dosage unit with a suitable solvent (*e.g*., water, alcohol, etc.), and subsequently extracting the drug susceptible to abuse from the dissolved mixture for use in a solution suitable for intravenous injection to achieve euphoria or a "high" comparable in some cases to the "high" experienced when injecting illicit drugs such as heroin

There is a growing need for novel compositions to deter misuse, abuse and diversion of orally administered pharmaceutical dosage units including but not limited to immediate release, sustained release, extended release, and delayed release dosage units containing drugs susceptible to abuse.

### SUMMARY OF THE INVENTION

According to some embodiments, a therapeutic composition includes a drug susceptible to abuse and foam forming agents. The foam forming agents may include an effervescent mixture; a surfactant; and a combination of at least one high viscosity polymer and at least one low viscosity polymer. In some embodiments, the foam forming agents include a foam stabilizing agent which may be present in an amount of about 0.5 wt% to about 20 wt% relative to the effervescent mixture; or about 1 wt% to about 15 wt% relative to the effervescent mixture.

In some embodiments, the effervescent mixture liberates gas in the presence of a media. In some embodiments, the effervescent mixture includes an organic acid and a salt. In some embodiments, the organic acid includes citric acid. In some embodiments, the salt includes alkaline bicarbonate. In certain embodiments, the stoichiometric ratio of organic acid to salt is 1:1. In some embodiments, the effervescent mixture is present in an amount of about 10 wt% to about 50 wt%.

In certain embodiments, the surfactant has a high hydrophilic/lipophilic balance. The surfactant may include sodium lauryl sulfate, and in some embodiments the surfactant is present in an amount of about 1 wt% to about 10 wt%.

In some embodiments, the ratio of a high viscosity polymer to a low viscosity polymer is about 5:1 to about 1:3, or about 3:1 to about 1:1. In certain embodiments, the combination of polymers is present in an amount of about 25 wt% to about 200 wt% relative to the effervescent mixture, or about 50 wt% to about 150 wt% relative to the effervescent mixture.

In some embodiments, the composition is in dosage unit(s) including but not limited to tablets or capsules.

### DESCRIPTION OF THE INVENTION

The present invention includes an abuse deterrent composition of ingredients for reducing the potential for misuse, abuse and diversion via one or more of (a) intravenous injection of dissolved dosage units, b) nasal snorting of crushed dosage units, and/or c) oral abuse via chewing whole dosage units, or swallowing excess quantities of dosage units.

In one embodiment, the present invention deters I.V. and/or nasal misuse and abuse by providing a pharmaceutical composition including one or more therapeutically active drugs susceptible to abuse, with one or more foam forming agents such that upon contact with solvents or wet nasal mucosal tissue, the agents generate a foam thereby increasing the difficulty of I.V. injection and nasal snorting.

Compositions and methods of some embodiments of the present invention relate to dosage units, such as orally administered pharmaceutical products, which contain one or more active pharmaceutical ingredients susceptible to abuse such that the resulting dosage unit is abuse deterrent. A composition of the present invention may contain a complement of ingredients that when subjected to water, aqueous solutions including hydroalcoholic solutions, and/or contacted with wet nasal mucosal tissues will generate a foam which will deter the extraction of the drug from the dosage unit or ability to inject the drug susceptible to abuse via I.V. and nasal routes of administration. A composition of the invention may contain an effervescent mixture which may liberate gas in the presence of water or other media which would facilitate the liberation of the gas, surfactant (or surfactant combinations), and water/pH dependent soluble polymers (or polymer combinations thereof). Compositions of the present invention may include immediate release, modified release, extended release, or delayed release oral solid dosage units such as tablets and capsules, quick dissolve dose units, sublingual tablets, buccal tablets, suppositories, pellets, effervescent preparations, soft chew and/or chewable tablets

### A. Constituents of an Abuse Deterrent Dosage Unit

Constituents of the present invention typically include one or more of 1) a drug suitable for use in the present invention; 2) a foam forming agent or agents; and 3) other constituents.

### 1. Drugs Suitable for Use with the Present Invention

Any drug, or therapeutically acceptable drug salt, drug derivative, drug analog, drug homologue, isomer, or polymorphs thereof may be used in the present invention. In one embodiment, the drug is formulated into an orally administered dosage unit. In certain embodiments, drugs susceptible to abuse are used. Drugs susceptible to abuse include but are not limited to psychoactive drugs, opioid analgesics, stimulants, tranquilizers, and other drugs that may cause euphoria and/or psychological and/or physical dependence.

In some embodiments, a drug for use in the present invention can be susceptible to abuse including but not limited to one or more of the following: alfentanil, alphaprodine, amphetamines, benzylmorphine, buprenorphine, butorphanol, carfentanil, clonitazene, codeine, desomorphine, dextromoramide, dextropropoxyphene, dezocine, dihydrocodeine, dihydromorphine, diphenoxylate, diprenorphine, etorphine, ethylmorphine, fentanyl, hydrocodone, hydromorphone, ohmefentanyl, levomethadryl, levomethadone, levorphanol, lofentanil, meperidine, methadone, methylmorphine, morphine, nalbuphine, nicomorphine, normorphine, normethadone, norpipanone, oxycodone, oxymorphone, pentazocine, pethidine, propoxyphene, remifentanil, sufentanil, tapentadol, tilidine, tramadol, and the coagulated juice of the plants belonging to the species Papaver somniferum (including the subspecies setigerum), including salts, derivatives, analogs, homologues, polymorphs thereof, and mixtures of any of the foregoing.

In some embodiments, the drug for use in the present invention can include norpseudoephedrine, amphetamine, methamphetamine, ephedrine, pseudoephedrine, pseudoephedrine HCl, pseudoephedrine sulfate, phenylpropanolamine, and methylphenidate or combinations thereof.

In some embodiments, a drug for use with the present invention which can be susceptible to abuse includes one or more of the following: allobarbital, allylprodine, alprazolam, amphetamine, amphetaminil, amobarbital, anileridine, barbital, bezitramide, bromazepam, diazepine, brotizolam, butobarbital, camazepam, cathine/D-norpseudoephedrine, chlordiazepoxide, clobazam, clonazepam, clorazepate, clotiazepam, cloxazolam, cyclobarbital, cyclorphan, cyprenorphine, delorazepam, diampromide, diazepam, dihydromorphine, dimenoxadol, dimephetamol, dimethylthiambutene, dioxaphetyl butyrate, dipipanone, dronabinol, eptazocine, estazolam, ethylloflazepate, etonitrazene, fencamfamine, fenethylline, fenproporex, fludiazepam, flunitrazepam, flurazepam, halazepam, haloxazolam, hydroxypethidine, isomethadone, hydroxymethylmorphinan, ketazolam, ketobemidone, loprazolam, lormetazepam, mazindol, medazepam, meprobamate, meptazinol, metazocine, methaqualone, methylphenobarbital, methyprylon, metopon, midazolam, modafinil, myrophine, narceine, nimetazepam, nordazepam, norlevorphenol, oxazepam, oxazolam, plants and plant parts of the plants belonging to the species Papaver somniferum, papaveretum, pernoline, pentobarbital, phenadoxone, phenomorphan, phenazocine, phenoperidine, piminodine, pholcodeine, phenmetrazine, phentermine, pinazepam, piritramide, prazepam, profadol, proheptazine, promedol, properidine, secbutabarbital, secobarbital, temazepam, tetrazepam, triazolam, vinylbital, each optionally in the form of corresponding stereoisomeric compounds and corresponding derivatives, including esters, ethers, salts and solvates..

In some embodiments, a drug may be present in a therapeutic composition in an amount of about 1 wt% to about 20 wt%; about 1 wt% to about 18 wt%; about 1 wt% to about 16 wt%; about 1 wt% to about 14 wt%; about 1 wt% to about 12 wt%; about 2 wt% to about 10 wt%; about 2 wt% to about 8 wt%; about 3 wt% to about 8 wt%; about 4 wt% to about 7 wt%; about 5 wt% to about 7 wt%, or about 6 wt% to about 7 wt%. In some embodiments, a drug may be present in a therapeutic composition in an amount of about 1 wt%; about 1.5 wt%; about 2 wt%; about 2.5 wt%; about 3 wt%; about 3.5 wt%; about 4 wt%; about 4.5 wt%; about 5 wt%; about 5.5 wt%; about 6 wt%; about 6.5 wt%; about 7 wt%; about 7.5 wt%; about 8 wt%; about 8.5 wt%; about 9 wt%; about 9.5 wt%; about 10 wt%; about 10.5 wt%; about 11 wt%; about 11.5 wt%; about 12 wt%; about 12.5 wt%; about 13 wt%; about 13.5 wt%; about 14 wt%; about 14.5 wt%; about 15 wt%; about 15.5 wt%; about 16 wt%; about 16.5 wt%; about 17 wt%; about 17.5 wt%; about 18 wt%; about 18.5 wt%; about 19 wt%; about 19.5 wt%; or about 20 wt%. In some embodiments, a drug may be present in a therapeutic composition in an amount of about 6.12 wt%.

In some embodiments, a drug is present in a therapeutic composition in an amount of about 1 mg, about 2 mg, about 3 mg, about 4 mg, about 5 mg, about 6 mg, about 7 mg, about 8 mg, about 9 mg, about 10 mg, about 11 mg, about 12, mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 m, about 45 mg, about 46 mg, about 47 mg, about 48 mg, about 49 mg, about 50 mg, about 55 mg, about 60 mg, about 65 mg, about 70 mg, about 75 mg, about 80 mg, about 85 mg, about 90 mg, about 95 mg, about 100 mg, about 125 mg, about 150 mg, about 175 mg, or about 200 mg.

In some embodiments, a pharmaceutical composition of the present invention includes one or more opioid analgesics such as hydrocodone, morphine, hydromorphone, codeine, and oxycodone and/or salts thereof, as the therapeutically active ingredient. Typically when processed into a suitable dosage unit, the drug can be present in such dosage units in an amount normally prescribed, typically about 0.5 to about 25 percent on a dry weight basis, based on the total weight of the dosage unit.

With respect to opioid analgesics dosage units, such an amount can be typically from about 0.1, 5, 25, 50, 75, 100, 125, 150, 175 or 200 mg. In some embodiments, the drug susceptible to abuse can be present in an amount from about 5 mg to about 500 mg or about 5 mg to about 200 mg. In some embodiments, a dosage unit contains an appropriate amount of drug to provide a therapeutic effect.

In some embodiments, the present invention includes one or more constituents which may or may not have pharmacological activity and which are not typically susceptible to abuse in addition to a drug which is susceptible to abuse, described above. In certain embodiments, the one or more constituents which are not typically susceptible to abuse can have an abuse deterrent effect (as described in more detail below) when administered in combination with a drug which is susceptible to abuse. In one embodiment of a dosage unit of the present invention which includes a drug that is susceptible to abuse, the one or more additional drugs which can induce an abuse deterrent effect can be included in the dosage unit in a sub-therapeutic or sub-clinical amount.

As used herein, "sub-therapeutic" or "sub-clinical" refer to an amount of a referenced drug substance that if consumed or otherwise administered, is insufficient to induce an abuse deterrent effect in atypical patient or is insufficient to meet or exceed the threshold dose necessary for inducing an abuse deterrent effect.

Accordingly, when an embodiment of a dosage unit of the present invention is administered in accordance with a health care provider prescribed dosage and/or manner, the one or more additional drugs which can induce an abuse deterrent effect will not be administered in an amount sufficient to induce an abuse deterrent effect. However, when a certain embodiment of the present invention is administered in a dose and/or manner that is different from a health care provider prescribed dose, (*i.e.,* excess oral doses are consumed as a single dose) the content of a dosage unit or multiple dosage units which can cause an abuse deterrent effect according to the present invention will be sufficient to induce an abuse deterrent effect. Suitable examples of drugs which can be administered in sub-therapeutic amounts in the present invention include niacin, atropine sulfate, homatropine methylbromide, sildenafil citrate, nifedipine, zinc sulfate, dioctyl sodium sulfosuccinate and capsaicin, as described in U.S. Patent Publication No. 2007/0231268, the entirety of which is incorporated herein by reference.

### 2. Foam Forming Agents

In certain embodiments, the invention includes a complement of ingredients which will generate a foam when subjected to (a) water; and/or (b) aqueous solutions including hydroalcoholic solutions; and/or (c) isotonic solutions. Such foam generation may deter the extraction and/or reduce the ability of the potential abuser to inject such solution via the intravenous route of administration. Such foam generation may also deter nasal abuse when the ingredients are contacted with moist human nasal mucosal tissue.

In certain embodiments, the complement of foam generating ingredients includes one or more of a) effervescent mixture; b) one or more surfactants; and c) polymers. In some embodiments, the complement of foam generating ingredients also includes foam stabilizing agents.

### a. Effervescent Mixture

Any suitable effervescent mixture which liberates gas in the presence of water or other media may be used. In some embodiments, an effervescent mixture includes the combination of an organic acid and a salt such as a bicarbonate or carbonate. Such a mixture may liberate carbon dioxide when mixed with a media. In some embodiments, an effervescent mixture includes the combination of an organic acid, such as citric acid, and alkaline bicarbonate, wherein carbon dioxide gas is rapidly liberated when the combination is mixed with water. In some embodiments, a mixture which produces rapid gas liberation improves the abuse deterrent properties of a composition of the present invention.

In some embodiments, an effervescent mixture includes a combination of one or more organic acids and bicarbonates/carbonates to produce sustained gas liberation. For example, a composition such as tartaric acid and calcium carbonate may liberate CO₂ more slowly than a combination of citric acid and alkaline bicarbonate due to lower water solubility. In some embodiments, a mixture which produces sustained gas liberation improves the abuse deterrent properties of a composition of the present invention.

In some embodiments, an effervescent mixture includes a combination of an organic acid and a carbonate/bicarbonate salt with an acid/salt ratio which is stoichiometrically about 1:1 for reactants. In some embodiments, an effervescent mixture includes a combination of an organic acid and a carbonate/bicarbonate salt with an acid/salt ratio of about 1.5:1; about 1.4:1; about 1.3:1; about 1.2:1; about 1.1:1; about 1:1; about 1:1.1; bout 1:1.2: about 1:1.3; about 1:1.4; or about 1:1.5. In some embodiments, an effervescent mixture includes a combination of an organic acid and a carbonate/bicarbonate salt with an acid/salt ratio in a range of about 1.5:1 to about 1:1.5. In one embodiment, an effervescent mixture includes 1 part citric acid to 1.3 parts sodium bicarbonate.

A composition of some embodiments of the present invention includes an effervescent mixture in an amount of about 5 wt% to about 75 wt%; about 5 wt% to about 70 wt%; about 5 wt% to about 65 wt%; about 10 wt% to about 60 wt%; about 15 wt% to about 55 wt%; about 10 wt% to about 50 wt%; about 15 wt% to about 45 wt%; about 20 wt% to about 40 wt%; about 25 wt% to about 35 wt%; or about 20 wt% to about 30 wt%. A composition of some embodiments of the present invention includes an effervescent mixture in an amount of about 5 wt%; about 10 wt%; about 15 wt%; about 20 wt%; about 25 wt%; about 30 wt%; about 35 wt%; about 40 wt%; about 45 wt%; about 50 wt%; about 55 wt%; about 60 wt%; about 65 wt%; about 70 wt%; or about 75 wt%.

### b. Surfactants

In some embodiments of the present invention, a composition includes one or more surfactants. Surfactants for use in compositions of the present invention can be selected from a wide range of surfactants used in the pharmaceutical industry. In some embodiments, suitable surfactants have a high hydrophilic/lipophilic balance (HLB) rating. High HLB value may be understood to mean an HLB value of equal to or greater than 10. Sodium lauryl sulfate is an example of a suitable high HLB surfactant. Other suitable surfactants may include but are not limited to polyoxyethylene sorbitan fatty acid esters such as but not limited to Polysorbate 20 and Polysorbate 80, polyoxyethylene castor oil derivatives such as but not limited to Polyoxyl 40 castor oil and Polyoxyl 60 hydrogenated castor oil, polyoxyethylene alkyl ethers such as but not limited to Cremaphor A 20 polyether and Ethylan 2560, polyoxyethylene stearates such as but not limited to Polyoxyl 100 stearate and Polyoxyl 150 distearate, polyoxylglycerides such as but not limited to lauroyl polyoxyglycerides and stearoyl polyoxyglycerides, poloxamers such as but not limited to Poloxamer 188, sucrose fatty acid esters such as but not limited to sucrose stearate and sucrose palmitate, phospholipids and docusate sodium. In some embodiments, a suitable surfactant lowers the surface tension of water such that the erupting bubbles of an effervescent mixture when subjected to a media are stabilized and foam is formed.

A composition of some embodiments of the present invention includes one or more surfactants in an amount of about 0.1 wt%; about 0.2 wt%; about 0.3 wt%; about 0.4 wt%; about 0.5 wt%; about 0.6 wt%; about 0.7 wt%; about 0.8 wt%; about 0.9 wt% about 1 wt%; about 2 wt%; about 3 wt%; about 4 wt%; about 5 wt%; about 6 wt%; about 7 wt%; about 8 wt%; about 9 wt%; about 10 wt%; about 11 wt%; about 12 wt%; about 13 wt%; about 14 wt%; about 15 wt%; about 16 wt%; about 17 wt%; about 18 wt%; about 19 wt%; or about 20 wt%. In some embodiments, a composition of the present invention includes one or more surfactants in an amount of about 0.1 wt% to about 20 wt%; about 0.2 wt% to about 19 wt%; about 0.3 wt% to about 18 wt%; about 0.4 wt% to about 17 wt%; about 0.5 wt% to about 16 wt%; about 0.6 wt% to about 15 wt%; about 0.7 wt% to about 14 wt%; about 0.8 wt% to about 13 wt%; about 0.9 wt% to about 12 wt%; about 1 wt% to about 11 wt%; about 0.1 wt% to about 10 wt%; about 0.2 wt% to about 9 wt%; about 0.3 wt% to about 8 wt%; about 0.4 wt% to about 7 wt%; or about 0.5 wt% to about 6 wt%.

### c. Polymers

Compositions of certain embodiments of the present invention include soluble polymers. Suitable soluble polymers may include water soluble polymers and pH dependent soluble polymers. Examples of suitable water soluble polymers include but are not limited to copovidone, methylcellulose, carbomer, carboxymethylcellulose sodium, ceratonia; gelatin, guar gum, hydroxyethyl cellulose, hydroxyethylmethyl cellulose, hydroxypropyl cellulose, hypromellose, methylcellulose polyethylene oxide, povidone, sodium hyaluronate, and xanthan gum. Examples of suitable pH dependent soluble polymers include but are not limited to sodium alginate, hypromellose acetate succinate, hypromellose phthalate, cellulose acetate phthalate, chitosan, polymethacrylates such as but not limited to poly(butyl metacrylate, (2-dimethylyaminoethyly) methacrylate, methyl methacrylate) and poly(methacrylicacid, ethylacrylate), and poly(methyl vinyl ether/maleic acid).

Inclusion of suitable polymers in compositions of the present invention may enhance certain desirable characteristics, including prolonging stabilized foam. For example, in some embodiments, polymer films in bubble formation add structure to in-situ generated foams.

In some embodiments, an abuse deterrent composition of the present invention may include a low viscosity polymer, a high viscosity polymer, and combinations thereof. Current grades are marked by viscosity which is a direct measure of molecular weight. Viscosity ranges for low viscosity polymers can range from about 3 to about 300 cps for 2% solution in water and from about 3,000 to about 100,000 cps for 2% solution in water for high viscosity polymers.

In some embodiments, a combination of a low viscosity polymer and a high viscosity polymer provide advantageous foam forming and stabilizing effects. In some embodiments, lower viscosity polymer can be important in the initial stages of foam formation. Without wishing to be bound by theory, low viscosity polymers may exhibit rapid hydration and gellation upon contact with a suitable media, and may therefore entrap gas into the foam as the CO₂ or other gas is instantaneously emitted upon contact with the media.

Again, without wishing to be bound by theory, as the early foaming process is occurring, a higher viscosity polymer may be more slowly hydrating and gelling and may then begin to contribute to the in-situ foaming process.

In some embodiments, a combination of a low viscosity polymer and a high viscosity polymer results in increased foam volume and prolonged foam stability in-situ foam as compared to low viscosity polymer or high viscosity polymer used individually. Without wishing to be bound by theory, when used alone, lower viscosity polymer may result in rapid in-situ foam generation with significant volume, however, over a short period of time (i.e. within 0.5-1 hr) the foam has a tendency to collapse. Without wishing to be bound by theory, a high viscosity polymer, when used alone in foam generation, may generate stable foams by adding structure to the foam bubbles, but the volume of the foam may be diminished.

In some embodiments, a composition includes a ratio of a first viscosity polymer to a second viscosity polymer in a range of about 5:1 to about 1:3; about 4:1 to about 1:2; about 3:1 to about 1:1; or about 2:1 to about 1:1. In some embodiments, a composition includes a ratio of a first viscosity polymer to a second viscosity polymer of about 5:1; about 4:1; about 3:1; about 2:1; about 1:1; about 1:2; about 1:3; about 1:2; about 1:3; about 1:4; or about 1:5. In some embodiments, the first viscosity polymer has a viscosity that is higher than the second viscosity polymer. In certain embodiments, the first viscosity polymer has a viscosity that is lower than the second viscosity polymer. In some embodiments, the first viscosity polymer is a high viscosity polymer, and the second viscosity polymer is a low viscosity polymer. In some embodiments, the first viscosity polymer is a low viscosity polymer, and the second viscosity polymer is a high viscosity polymer.

In some embodiments, a composition may include a combination of a low and a high viscosity polymer from the same polymer class, such as but not limited to hypromellose, povidone, or polyethylene oxide. In some embodiments, a combination of two different polymers, one of low viscosity and the other of high viscosity, includes polyethylene oxide, NF and hypromellose NF. Other polymer combinations include but are not limited to povidone NF and hypromellose NF, polyethylene oxide, NF and povidone USP, hypromellose and hydroxypropyl cellulose NF, hydroxypropylcellulose NF and polyethylene oxide NF, hydroxypropylcellulose NF and povidone USP, methylcellulose USP and povidone USP, and polyethylene oxide NF and methylcellulose USP.

In some embodiments, a composition of the present invention includes one or more polymers in an amount of about 25 wt% to about 200 wt% relative to the effervescent mixture; about 25 wt% to about 175 wt% relative to the effervescent mixture; about 30 wt% to about 170 wt% relative to the effervescent mixture; about 35 wt% to about 165 wt% relative to the effervescent mixture; about 40 wt% to about 160 wt% relative to the effervescent mixture; about 45 wt% to about 155 wt% relative to the effervescent mixture; about 50 wt% to about 150 wt% relative to the effervescent mixture; about 55 wt% to about 145 wt% relative to the effervescent mixture; about 60 wt% to about 140 wt% relative to the effervescent mixture; about 65 wt% to about 135 wt% relative to the effervescent mixture; about 70 wt% to about 130 wt% relative to the effervescent mixture; about 75 wt% to about 125 wt% relative to the effervescent mixture; about 80 wt% to about 120 wt% relative to the effervescent mixture; about 85 wt% to about 115 wt% relative to the effervescent mixture; about 90 wt% to about 110 wt% relative to the effervescent mixture; or about 95 wt% to about 105 wt% relative to the effervescent mixture. In some embodiments, a composition of the present invention includes one or more polymers in an amount of about 25 wt% relative to the effervescent mixture; about 30 wt% relative to the effervescent mixture; about 35 wt% relative to the effervescent mixture; about 40 wt% relative to the effervescent mixture; about 45 wt% relative to the effervescent mixture; about 50 wt% relative to the effervescent mixture; about 55 wt% relative to the effervescent mixture; about 60 wt% relative to the effervescent mixture; about 65 wt% relative to the effervescent mixture; about 70 wt% relative to the effervescent mixture; about 75 wt% relative to the effervescent mixture; about 80 wt% relative to the effervescent mixture; about 85 wt% relative to the effervescent mixture; about 90 wt% relative to the effervescent mixture; about 95 wt% relative to the effervescent mixture; about 100 wt% relative to the effervescent mixture; about 105 wt% relative to the effervescent mixture; about 110 wt% relative to the effervescent mixture; about 115 wt% relative to the effervescent mixture; about 120 wt% relative to the effervescent mixture; about 125 wt% relative to the effervescent mixture; about 130 wt% relative to the effervescent mixture; about 135 wt% relative to the effervescent mixture; about 140 wt% relative to the effervescent mixture; about 145 wt% relative to the effervescent mixture; about 150 wt% relative to the effervescent mixture; about 155 wt% relative to the effervescent mixture; about 160 wt% relative to the effervescent mixture; about 165 wt% relative to the effervescent mixture; about 170 wt% relative to the effervescent mixture; about 175 wt% relative to the effervescent mixture; about 180 wt% relative to the effervescent mixture; about 185 wt% relative to the effervescent mixture; about 190 wt% relative to the effervescent mixture; about 195 wt% relative to the effervescent mixture; or about 200 wt% relative to the effervescent mixture.

### d. Foam Stabilizing Agents

Compositions of certain embodiments of the present invention may include foam stabilizing agents, which may prevent or retard the coalescence of gas bubbles. As a result, foam stabilizing agents may increase the time or duration that the resultant foam will have abuse deterrent features. In some embodiments of this invention, foam stabilizing agents act to prolong the resultant foam in the presence of volumetric additions of water or other solvents and to resist a collapse of the initial foam formation, thus maintaining the deterrence to injection for an extended period. Suitable foam stabilizing agents may include but are not limited to agar, beta glucan, carrageenan, gelatin, hydrophobin class II (HFBII), lecithin, and sucrose surfactants. A preferred embodiment of the invention may include hydrophobin class II (HFBII).

In some embodiments, a composition of the present invention includes one or more foam stabilizering agents in an amount of about 0.1 wt% to about 30 wt% relative to the effervescent mixture; about 0.1 wt% to about 28 wt% relative to the effervescent mixture; about 0.1 wt% to about 26 wt% relative to the effervescent mixture; about 0.1 wt% to about 24 wt% relative to the effervescent mixture; about 0.1 wt% to about 22 wt% relative to the effervescent mixture; about 0.5 wt% to about 20 wt% relative to the effervescent mixture; about 1 wt% to about 18 wt% relative to the effervescent mixture; about 1 wt% to about 16 wt% relative to the effervescent mixture; about 1 wt% to about 15 wt% relative to the effervescent mixture; about 2 wt% to about 14 wt% relative to the effervescent mixture; about 0.1 wt% relative to the effervescent mixture; about 0.2 w% relative to the effervescent mixture; about 0.3 wt% relative to the effervescent mixture; about 0.4 wt% relative to the effervescent mixture; about 0.5 wt% relative to the effervescent mixture; about 0.6 wt% relative to the effervescent mixture; about 0.7 wt% relative to the effervescent mixture; about 0.8 wt% relative to the effervescent mixture; about 0.9 wt% relative to the effervescent mixture; about 1 wt% relative to the effervescent mixture; about 2 wt% relative to the effervescent mixture; about 4 wt% relative to the effervescent mixture; about 6 wt% relative to the effervescent mixture; about 8 wt% relative to the effervescent mixture; about 10 wt% relative to the effervescent mixture; about 12 wt% relative to the effervescent mixture; about 14 wt% relative to the effervescent mixture; about 16 wt% relative to the effervescent mixture; about 18 wt% relative to the effervescent mixture; about 20 wt% relative to the effervescent mixture; about 22 wt% relative to the effervescent mixture; about 24 wt% relative to the effervescent mixture; about 26 wt% relative to the effervescent mixture; about 28 wt% relative to the effervescent mixture; or about 30 wt% relative to the effervescent mixture.

### e. Media

In some embodiments, a composition of the present invention includes an effervescent mixture which liberates gas in the presence of media which would facilitate the liberation of the gas, *e.g.* water. Media may include, but is not limited to, aqueous solutions such as hydroalcoholic solutions, where ethanol may be the primary alcoholic agent and hydroalcoholic combinations from 1 part to 95 parts alcohol in water, buffered solutions with pH modifiers such as but not limited to citrates, acetates, phosphate, ammoniates, borates, and glycine, isotonic solutions such as but not limited to sodium chloride and dextrose, and human and animal fluids such as but not limited to blood, serum, plasma and nasal mucosal fluids.

### 3. Additional Constituents

The present invention can also optionally include other ingredients to enhance dosage unit manufacture from a pharmaceutical composition of the present invention and/or alter the release profile of a dosage unit including a pharmaceutical composition of the present invention, including but not limited to fillers, disintegrants, glidants, and lubricants.

### a. Fillers

Some embodiments of the present invention include one or more pharmaceutically acceptable fillers/diluents. In some embodiments, a therapeutic composition includes any suitable binder or filler. In some embodiments, a therapeutic composition includes microcrystalline cellulose. In some embodiments, suitable microcrystalline cellulose can have an average particle size ranging from about 20 to about 200 µm, and in some embodiments about 100 µm. In some embodiments, the density ranges from about 1.512 to about 1.668 g/cm³. Other ingredients can include sugars and/or polyols.

In some embodiments, a therapeutic composition includes microcrystalline cellulose in an amount of about 20 wt% to about 35 wt%; about 22 wt% to about 32 wt%; about 24 wt% to about 30 wt%; or about 26 wt% to about 28 wt%. In some embodiments, a therapeutic composition includes microcrystalline cellulose in an amount of about 20 wt%; about 21 wt%; about 22 wt%; about 23 wt%; about 24 wt%; about 25 wt%; about 26 wt%; about 27 wt%; about 28 wt%; about 29 wt%; about 30 wt%; about 31 wt%; about 32 wt%; about 33 wt%; about 34 wt%; or about 35 wt%.

In certain embodiments; a therapeutic composition includes microcrystalline cellulose in an amount of about 100 mg to about 160 mg; about 105 mg to about 155 mg; about 110 mg to about 150 mg; about 115 mg to about 145 mg; about 120 mg to about 140 mg; about 125 mg to about 135 mg; or about 120 mg to about 135 mg. In certain embodiments, a therapeutic composition includes microcrystalline cellulose in an amount of about 100 mg; about 105 mg; about 110 mg; about 115 mg; about 120 mg; about 125 mg; about 130 mg; about 135 mg; about 140 mg; about 145 mg; about 150 mg; or 155 mg.

In some embodiments of the invention, the fillers which can be present at about 10 to 65 percent by weight on a dry weight basis, also function as binders in that they not only impart cohesive properties to the material within the formulation, but can also increase the bulk weight of a directly compressible formulation to achieve an acceptable formulation weight. In some embodiments, additional fillers need not provide the same level of cohesive properties as the binders selected, but can be capable of contributing to formulation homogeneity and resist segregation from the formulation once blended. Further, preferred fillers do not have a detrimental effect on the flowability of the composition or dissolution profile of the formed tablets.

### b. Disintegrants

In some embodiments, the present invention can include one or more pharmaceutically acceptable disintegrants. Such disintegrants are known to a skilled artisan. In some embodiments, a therapeutic composition includes crospovidone (such as Polyplasdone® XL) having a particle size of about 400 microns and a density of about 1.22 g/ml. In some embodiments, disintegrants can include, but are not limited to, sodium starch glycolate (Explotab®) having a particle size of about 104 microns and a density of about 0.756 g/ml, starch (*e.g*., Starch 21) having a particle size of about 2 to about 32 microns and a density of about 0.462 g/ml, and croscarmellose sodium (Ac-Di-Sol) having a particle size of about 37 to about 73.7 microns and a density of about 0.529 g/ml. The disintegrant selected should contribute to the compressibility, flowability and homogeneity of the formulation. Further the disintegrant can minimize segregation and provide an immediate release profile to the formulation. An immediate release drug product is understood in the art to allow drugs to dissolve with no intention of delaying or prolonging dissolution or absorption of the drug upon administration, as opposed to products which are formulated to make the drug available over an extended period after administration. In some embodiments, the disintegrant(s) are present in an amount from about 2 wt% to about 25 wt%.

In some embodiments, a therapeutic composition includes crospovidone in an amount of about 15 wt% to about 25 wt%; about 18 wt% to about 22 wt%; or about 19 wt% to about 21 wt%. In some embodiments, a therapeutic composition includes crospovidone in an amount of about 15 wt%; about 16 wt%; about 17 wt%; about 18 wt%; about 19 wt%; about 20 wt%; about 21 wt%; about 22 wt%; about 23 wt%; about 24 wt%; or about 25 wt%.

In some embodiments, a therapeutic composition includes crospovidone in an amount of about 75 mg to about 125 mg; about 80 mg to about 120 mg; about 85 mg to about 115 mg; about 90 mg to about 110 mg; or about 95 mg to about 105 mg. In some embodiments, a therapeutic composition includes crospovidone in an amount of about 75 mg; about 80 mg; about 85 mg; about 90 mg; about 95 mg; about 100 mg; about 105 mg; about 110 mg; about 115 mg; about 120 mg; or about 125 mg.

### c. Glidants

In one embodiment, the present invention can include one or more pharmaceutically acceptable glidants, including but not limited to colloidal silicon dioxide. In one embodiment, colloidal silicon dioxide (Cab-O-Sil®) having a density of about 0.029 to about 0.040 g/ml can be used to improve the flow characteristics of the formulation. Such glidants can be provided in an amount of from about 0.1 wt% to about 1 wt%; about 0.2 wt% to about 0.8 wt%; or about 0.2 to about 6 wt%. In some embodiments, a therapeutic composition includes a glidant in an amount of about 0.1 wt%; about 0.2 wt%; about 0.3 wt%; about 0.4 wt%; about 0.5 wt%; about 0.6 wt%; about 0.7 wt%; about 0.8 wt%; about 0.9 wt%; or about 1 wt%. In some embodiments, a therapeutic composition includes a glidant in an amount of about 0.1 mg to about 10 mg; about 0.5 mg to about 10 mg; about 1 mg to about 10 mg; about 1 mg to about 5 mg; or about 1 mg to about 3 mg. In some embodiments, a therapeutic composition includes a glidant in an amount of about 0.1 mg; about 0.5 mg; about 1 mg; about 2 mg; about 3 mg; about 4 mg; about 5 mg; about 6 mg; about 7 mg; about 8 mg; about 9 mg; or about 10 mg.

It will be understood, based on this invention, however, that while colloidal silicon dioxide is one particular glidant, other glidants having similar properties which are known or to be developed could be used provided they are compatible with other excipients and the active ingredient in the formulation and which do not significantly affect the flowability, homogeneity and compressibility of the formulation.

### d. Lubricants

In one embodiment, the present invention can include one or more pharmaceutically acceptable lubricants, including but not limited to magnesium stearate. In some embodiments, magnesium stearate has a particle size of about 450 to about 550 microns and a density of about 1.00 to about 1.80 g/ml. In some embodiments of the present invention, a therapeutic composition includes magnesium stearate having a particle size of from about 5 to about 50 microns and a density I of from about 0.1 to about 1.1 g/ml. In certain embodiments, magnesium stearate can contribute to reducing friction between a die wall and a pharmaceutical composition of the present invention during compression and can ease the ejection of the tablets, thereby facilitating processing. In some embodiments, the lubricant resists adhesion to punches and dies and/or aid in the flow of the powder in a hopper and/or into a die. In some embodiments, suitable lubricants are stable and do not polymerize within the formulation once combined. Other lubricants which exhibit acceptable or comparable properties include stearic acid, hydrogenated oils, sodium stearyl fumarate, polyethylene glycols, and Lubritab®.

In certain embodiments, a therapeutic composition includes lubricant in an amount of about 0.1 wt% to about 5 wt%; about 0.1 wt% to about 3 wt%; about 0.1 wt% to about 1 wt%; or about 0.1 wt% to about 0.5 wt%. In some embodiments, a therapeutic composition includes lubricant in an amount of about 0.1 wt%; about 0.2 wt%; about 0.3 wt%; about 0.4 wt%; about 0.5 wt%; about 0.6 wt%; about 0.7 wt%; about 0.8 wt%; about 0.9 wt%; or about 1 wt%. In some embodiments, a therapeutic composition includes lubricant in an amount of about 0.5 mg to about 5 mg; about 0.5 mg to about 3 mg; or 0.5 mg to about 1.5 mg. In some embodiments, a therapeutic composition includes lubricant in an amount of about 0.5 mg; about 1 mg; about 1.5 mg; about 2 mg; about 2.5 mg; about 3 mg; about 4 mg; about 5 mg; about 6 mg; about 7 mg; about 8 mg; about 9 mg; or about 10 mg.

In certain embodiments, the most important criteria for selection of the excipients are that the excipients should achieve good content uniformity and release the active ingredient as desired. The excipients, by having excellent binding properties, and homogeneity, as well as good compressibility, cohesiveness and flowability in blended mixtures, minimize segregation of powders in the hopper during direct compression.

### 4. Methods of Making

In some embodiments, any of the constituents may or may not be sequestered from the other constituents during the manufacturing or in the final dosage units(*e.g*., tablet or capsule). In some embodiments, one or more of the constituents may be sequestered. In some embodiments, one or more of the constituents is blended and/or admixed such that all or a portion of the constituents are in contact with other constituents and/or are not sequestered.

A pharmaceutical composition of the present invention including one or more drug, an effervescent mixture, one or more surfactant, and one or more polymer, can be suitably modified and processed to form a dosage unit of the present invention. In this manner, an abuse deterrent composition can be layered onto, coated onto, applied to, admixed with, formed into a matrix with, and/or blended with a drug and optionally other ingredients, thereby providing a therapeutic composition of the present invention. The dosage units can be any shape, including regular or irregular shape depending on the needs of the artisan.

Compressed tablets including the pharmaceutical compositions of the present invention can be direct compression tablets. In some embodiments, a dosage unit of the present invention can be made by wet granulation, and dry granulation (*e.g*. slugging, roller compaction, or hot melt extrusion). The method of preparation and type of excipients are selected to give the tablet dosage unit desired physical characteristics that allow for rapid compression of the tablets. After compression, the tablets must have a number of additional attributes such as appearance, hardness, disintegrating ability, and an acceptable dissolution profile. Tablets may be further film coated with various film coating materials such as but not limited to gelatin, hypromellose, hydroxyl propyl cellulose, and polymethacrylates based on uncoated tablet weight. Tablet coating may be applied by spray application, dipping, enrobing or any other practicable ways of applying coating to tablets. Tablet coating can be applied to improve aesthetic appearance of the tablets, taste mask the product, aid in swallowing or to delay the release of foam until the tablet has reached the stomach.

Choice of fillers and other excipients typically depend on the chemical and physical properties of the drug, behavior of the mixture during processing, and the properties of the final tablets. Adjustment of such parameters is understood to be within the general understanding of one skilled in the relevant art. Suitable fillers and excipients are described in detail above.

The manufacture of a dosage unit of the present invention can involve direct compression and wet and dry granulation methods, including slugging, roller compaction, high shear granulation and fluid bed granulation. In some embodiments, it is preferred to use direct compression techniques because of the lower processing time and cost advantages as well as a controlled dry process for the effervescent product.

Accordingly, and as described further below, a directly compressible pharmaceutical composition of the present invention can be designed following the teachings set forth herein that can deter one or more of a) I.V. abuse of a dissolved dosage unit; b) nasal snorting of a crushed dosage unit; c) oral abuse via swallowing excess quantities of a dosage unit and d) conversion of a dosage unit for potential abuse using illicit processes.

Steps for making the compositions or dosage units include the step of providing one or more drugs described above and foam forming agents including an effervescent mixture, one or more surfactants, and one or more polymers as described above and/or providing a disintegrant and other ingredients in the amounts described above.

By controlling the amounts of the constituents relative to each other, and the viscosities of the polymers, a therapeutic composition suitable for use to deter drug abuse can be formed. In some embodiments, in addition to the routes of abuse noted above, a composition according to the present invention inhibits the conversion of one drug or precursor compound into a drug susceptible to abuse.

### B. Abuse Deterrence

Drug abusers may attempt to manipulate an oral solid pharmaceutical dosage unit containing one or more drugs susceptible to abuse by crushing, shearing, grinding, chewing, dissolving heating, extracting or otherwise tampering with or damaging the dosage unit so that a significant portion or even the entire amount of the active drug(s) in the dosage unit become available for misuse, abuse and diversion by 1) injection of dissolved dosage unit(s), 2) nasal snorting of crushed dosage units, and/or 3) oral swallowing excess quantities of dosage units This invention is designed to deter misuse and abuse by both injection and inhalation.

For I.V. abuse, the solid dose is typically ground or milled to a fine powder and a small amount (<2 mL) of a suitable solvent is added, mixed and the resulting liquid mixture is drawn into a syringe and injected in the bloodstream. However, when about 2 mL of water or other media is added to a composition of the present invention, stiff foam may be generated as described above. A significant amount, or all, of the media may be consumed in the formation of the foam. Such foam may be difficult or impossible to draw into a syringe. Additional small amounts of media (about 1-2 mL) may be added and the foam may continue to generate, with no solvent mixture volume available to be syringed. Addition of more media may transform the stiff foam to more fluid foam, however, due to the presence of viscous polymers, the fluid foam may be too viscous to be drawn into a hypodermic syringe. Adding additional media may allow the fluid foam to be drawn into a syringe; however, due to the presence of the stabilized gas bubbles still entrapped in the fluid foam, I.V. injection of the fluid foam is impractical or impossible.

For nasal abuse, the solid dosage unit is typically required to be ground into a fine powder such that it can be insufflated or snorted into the nasal cavity. The drug may be rapidly absorbed through the nasal mucosal membranes and may be rapidly transported across the blood brain barrier to the brain to elicit a rapid and substantial drug response or "high". However, a composition of some embodiments of the present invention may begin to form stiff foam when in the presence of the wet nasal mucosal membranes of the nasal cavity. As the volume of the foam increases in the nasal cavity, the subject may experience a cough/sneezing reflex to remove the foam from the nasal cavity. Thus the drug may be expelled from the nasal cavity and the absorption of the drug through the nasal membrane may be minimized.

As used herein, the term "about" is understood to mean ±10% of the value referenced. For example, "about 10 wt%" is understood to literally mean 9 wt% to 11 wt%.

A number of references have been cited, the entire disclosures of which are incorporated herein by reference.

### EXAMPLES

Therapeutic compositions were prepared according to the formulations of the following examples.

### Example 1

A 490 mg tablet was prepared according to the following formulation:

| **Component** | **Amount** |
|---|---|
| Oxycodone HCl | 5 mg |
| Niacin | 30 mg |
| Polyox™ WSR Coagulant | 110 mg |
| (nonionic poly (ethylene oxide) polymers) | |
| Sodium Bicarbonate | 66 mg |
| Methocel™ E6 | 55 mg |
| (hypromellose) | |
| Citric Acid, monohydrate | 54 mg |
| Ac-Di-Sol | 30 mg |
| (croscarmellose sodium) | |
| Sodium Lauryl Sulfate | 15 mg |
| Gelatin | 20 mg |
| Magnesium Stearate | 2.5 mg |
| Avicel PH 102 | q.s. 490 mg tablet |
| (microcrystalline cellulose) | |

The tablet was prepared by first blending all components except the tablet lubricant (magnesium stearate). The magnesium stearate was then added to the powder mixture and final blended. The final blend was compressed into tablets and the compressed tablets were coated with an appropriate film coating.

### Example 2

A 550 mg tablet was prepared according to the following formulation:

| **Component** | **Amount** |
|---|---|
| Oxycodone HCl | 7.5 mg |
| Niacin | 30 mg |
| Sodium Bicarbonate | 99 mg |
| Methocel™ K3 | 100 mg |
| (hypromellose) | |
| Citric Acid, monohydrate | 81 mg |
| Methocel™ K100M premium | 50 mg |
| (hypromellose) | |
| Explotab^{®} | 50 mg |
| (sodium starch glycolate and sodium carboxymethyl starch) | |
| Sucrose stearate | 25 mg |
| Magnesium Stearate | 3 mg |
| Cab-O-Sil^{®} | 1 mg |
| (silica) | |
| Avicel PH 103 | q.s. 550 mg tablet |
| (microcrystalline cellulose) | |

The tablet was prepared by blending sodium bicarbonate, Methocel™ K3, citric acid monohydrate, Methocel™ K100 M premium, sucrose stearate and portions of Explotab^{®}, magnesium stearate and Avicel PH103. The intermediate blend was roller compacted and sized by milling to an intermediate granulation. The intermediate granulation was then final blended with the remaining components and the final blend was compressed into tablets.

### Example 3

A 475 mg tablet was prepared according to the following formulation:

| **Component** | **Amount** |
|---|---|
| Hydromorphone HCl | 4 mg |
| Potassium Bicarbonate | 100 mg |
| Plasdone^{®} K29/32 | 80 mg |
| (povidone) | |
| Tartaric Acid | 75 mg |
| Klucel^{®} MF | 60 mg |
| (hydroxypropylcellulose) | |
| Polyplasdone^{®} XL | 40 mg |
| (crospovidone) | |
| Sodium Lauryl Sulfate | 20 mg |
| Class II hydrophobin | 5 mg |
| Magnesium Stearate | 3 mg |
| Cab-O-Sil^{®} | 1 mg |
| (silica) | |
| Avicel PH 112 | q.s. 475 mg tablet |
| (microcrystalline cellulose) | |

Potassium bicarbonate, Plasdone^{®} K29/32, tartaric acid, Klucel^{®} MF, sodium lauryl sulfate, class II hydrophobin and portions of Polyplasdone^{®} XL and Avicel PH112 were wet granulated by high shear with denatured alcohol and dried to remove the alcohol. The intermediate granulation was final blended with the remaining components and the final blend was compressed into tablets. Compressed tablets were coated with an appropriate film coating.

### Example 4

A 525 mg tablet was prepared according to the following formulation:

| **Component** | **Amount** |
|---|---|
| Morphine Sulfate | 30 mg |
| Niacin | 30 mg |
| Methocel™ E15 Premium LV | 75 mg |
| (hypromellose) | |
| Polyox™WSR Coagulant | 40 mg |
| (nonionic poly(ethyleneoxide) polymers) | |
| Sodium carbonate, monohydrate | 31 mg |
| Citric acid, monohydrate | 35 mg |
| Ac-Di-Sol | 25 mg |
| (croscarmellose sodium) | |
| Explotab^{®} | 25 mg |
| (sodium starch glycolate and sodium carboxymethyl starch) | |
| Sucrose palmitate | 22.5 mg |
| Class II Hydrophobin | 3.5 mg |
| Magnesium Stearate | 2.5 mg |
| Avicel PH 112 | q.s. ad 525 mg tablet |
| Lactose anhydrous | |

Sodium carbonate, monohydrate, Methocel^{®} E 15 Premium LV, Polyox™ WSR Coagulant, citric acid monohydrate, sucrose palmitate, Class II hydrophobin and a portion of Explotab^{®} were hot melt extruded, cooled and sized to an intermediate granulation. The intermediate granulation was final blended with the remaining components and the final blend was compressed into tablets.

## Claims

1. A pharmaceutical composition comprising:
a. a drug susceptible to abuse; and
b. foam forming agents comprising
i. an effervescent mixture;
ii. a surfactant; and
iii. a combination of at least one high viscosity polymer and at least one low viscosity polymer.

2. The pharmaceutical composition of claim 1, wherein the effervescent mixture liberates gas in the presence of a media.

3. The pharmaceutical composition of claim 1, wherein the effervescent mixture comprises an organic acid and a salt.

4. The pharmaceutical composition of claim 3, wherein the organic acid comprises citric acid.

5. The pharmaceutical composition of claim 3, wherein the salt comprises alkaline bicarbonate.

6. The pharmaceutical composition of claim 3, wherein the stochiometric ratio of organic acid to salt is 1:1.

7. The pharmaceutical composition of clam 1, wherein the effervescent mixture is present in an amount of about 10 wt% to about 50 wt%.

8. The pharmaceutical composition of claim 1, wherein the surfactant has a high hydrophilic/lipophilic balance.

9. The pharmaceutical composition of claim 1, wherein the surfactant comprises sodium lauryl sulfate.

10. The pharmaceutical composition of claim 1, wherein the surfactant is present in an amount of about 1 wt% to about 10 wt%.

11. The pharmaceutical composition of claim 1, wherein the ratio of a high viscosity polymer to a low viscosity polymer is about 5:1 to about 1:3.

12. The pharmaceutical composition of claim 1, wherein the ratio of a high viscosity polymer to a low viscosity polymer is about 3:1 to about 1:1.

13. The pharmaceutical composition of claim 1, wherein the combination of polymers is present in an amount of about 25 wt% to about 200 wt% relative to the effervescent mixture.

14. The pharmaceutical composition of claim 1, wherein the combination of polymers is present in an amount of about 50 wt% to about 150 wt% relative to the effervescent mixture.

15. The pharmaceutical composition of claim 1, the foam forming agents further comprising a foam stabilizing agent.

16. The pharmaceutical composition of claim 15, wherein the foam stabilizing agent is present in an amount of about 0.5 wt% to about 20 wt% relative to the effervescent mixture.

17. The pharmaceutical composition of claim 15, wherein the foam stabilizing agent is present in an amount of about 1 wt% to about 15 wt% relative to the effervescent mixture.

18. The pharmaceutical composition of claim 1, wherein the composition is in unit dose form.

## Patentansprüche

1. Ein Arzneimittel, umfassend:
a. einen missbrauchsanfälligen Arzneistoff; und
b. Schaumbildner, umfassend
i. ein Brausegemisch;
ii. ein oberflächenaktives Mittel; und
iii. eine Kombination von mindestens einem Polymer mit hoher Viskosität und mindestens einem Polymer mit geringer Viskosität.

2. Das Arzneimittel nach Anspruch 1, wobei das Brausegemisch in Gegenwart eines Mediums Gas freigibt.

3. Das Arzneimittel nach Anspruch 1, wobei das Brausegemisch eine organische Säure und ein Salz umfasst.

4. Das Arzneimittel nach Anspruch 3, wobei die organische Säure Zitronensäure umfasst.

5. Das Arzneimittel nach Anspruch 3, wobei das Salz alkalisches Hydrogencarbonat umfasst.

6. Das Arzneimittel nach Anspruch 3, wobei das stöchiometrische Verhältnis von organischer Säure zu Salz 1:1 ist.

7. Das Arzneimittel nach Anspruch 1, wobei das Brausegemisch in einer Menge von etwa 10 Gew.-% bis etwa 50 Gew.-% vorhanden ist.

8. Das Arzneimittel nach Anspruch 1, wobei das oberflächenaktive Mittel einen hohen HLB-Wert (engl.: hydrophilic/lipophilic balance) aufweist.

9. Das Arzneimittel nach Anspruch 1, wobei das oberflächenaktive Mittel Natriumlaurylsulfat umfasst.

10. Das Arzneimittel nach Anspruch 1, wobei das oberflächenaktive Mittel in einer Menge von etwa 1 Gew.-% bis etwa 10 Gew.-% vorhanden ist.

11. Das Arzneimittel nach Anspruch 1, wobei das Verhältnis eines Polymers mit hoher Viskosität zu einem Polymer mit geringer Viskosität etwa 5:1 bis etwa 1:3 beträgt.

12. Das Arzneimittel nach Anspruch 1, wobei das Verhältnis eines Polymers mit hoher Viskosität zu einem Polymer mit geringer Viskosität etwa 3:1 bis etwa 1:1 beträgt.

13. Das Arzneimittel nach Anspruch 1, wobei die Kombination von Polymeren in einer Menge von etwa 25 Gew.-% bis etwa 200 Gew.-%, im Verhältnis zum Brausegemisch, vorhanden ist.

14. Das Arzneimittel nach Anspruch 1, wobei die Kombination von Polymeren in einer Menge von etwa 50 Gew.-% bis etwa 150 Gew.-%, im Verhältnis zum Brausegemisch, vorhanden ist.

15. Das Arzneimittel nach Anspruch 1, wobei die Schaumbildner ferner ein schaumstabilisierendes Mittel umfassen.

16. Das Arzneimittel nach Anspruch 15, wobei das schaumstabilisierende Mittel in einer Menge von etwa 0,5 Gew.-% bis etwa 20 Gew.-%, im Verhältnis zum Brausegemisch, vorhanden ist.

17. Das Arzneimittel nach Anspruch 15, wobei das schaumstabilisierende Mittel in einer Menge von etwa 1 Gew.-% bis etwa 15 Gew.-%, im Verhältnis zum Brausegemisch, vorhanden ist.

18. Das Arzneimittel nach Anspruch 1, wobei das Arzneimittel in Form von Dosierungseinheiten vorliegt.

## Revendications

1. Composition pharmaceutique comprenant :
a. un médicament susceptible d'un usage abusif ; et
b. des agents formant une mousse comprenant
i. un mélange effervescent ;
ii. un tensioactif ; et
iii. une combinaison d'au moins un polymère de viscosité élevée et d'au moins un polymère de faible viscosité.

2. Composition pharmaceutique selon la revendication 1, dans laquelle le mélange effervescent libère un gaz en présence d'un milieu.

3. Composition pharmaceutique selon la revendication 1, dans laquelle le mélange effervescent comprend un acide organique et un sel.

4. Composition pharmaceutique selon la revendication 3, dans laquelle l'acide organique comprend de l'acide citrique.

5. Composition pharmaceutique selon la revendication 3, dans laquelle le sel comprend un bicarbonate alcalin.

6. Composition pharmaceutique selon la revendication 3, dans laquelle le rapport stoechiométrique de l'acide organique au sel est de 1/1.

7. Composition pharmaceutique selon la revendication 1, dans laquelle le mélange effervescent est présent en une quantité d'environ 10 % en poids à environ 50 % en poids.

8. Composition pharmaceutique selon la revendication 1, dans laquelle le tensioactif a un équilibre hydrophile/lipophile élevé.

9. Composition pharmaceutique selon la revendication 1, dans laquelle le tensioactif comprend du laurylsulfate de sodium.

10. Composition pharmaceutique selon la revendication 1, dans laquelle le tensioactif est présent en une quantité d'environ 1 % en poids à environ 10 % en poids.

11. Composition pharmaceutique selon la revendication 1, dans laquelle le rapport du polymère de viscosité élevée au polymère de faible viscosité est d'environ 5/1 à environ 1/3.

12. Composition pharmaceutique selon la revendication 1, dans laquelle le rapport d'un polymère de viscosité élevée à un polymère de faible viscosité est d'environ 3/1 à environ 1/1.

13. Composition pharmaceutique selon la revendication 1, dans laquelle la combinaison de polymères est présente en une quantité d'environ 25 % en poids à environ 200 % en poids par rapport au mélange effervescent.

14. Composition pharmaceutique selon la revendication 1, dans laquelle la combinaison de polymères est présente en une quantité d'environ 50 % en poids à environ 150 % en poids par rapport au mélange effervescent.

15. Composition pharmaceutique selon la revendication 1, dans laquelle les agents formant une mousse comprennent en outre un agent stabilisateur de mousse.

16. Composition pharmaceutique selon la revendication 15, dans laquelle l'agent stabilisateur de mousse est présent en une quantité d'environ 0,5 % en poids à environ 20 % en poids par rapport au mélange effervescent.

17. Composition pharmaceutique selon la revendication 15, dans laquelle l'agent stabilisateur de mousse est présent en une quantité d'environ 1 % en poids à environ 15 % en poids par rapport au mélange effervescent.

18. Composition pharmaceutique selon la revendication 1, laquelle composition est sous une forme posologique unitaire.
